# NEW EUROPEAN PATENT SPECIFICATION

(11) **EP 0 531 506 B2**
(45) Date of publication and mention of the opposition decision: **21.05.2003**
(45) Mention of the grant of the patent: 16.12.1998
(21) Application number: 92909628.7
(22) Date of filing: 05.03.1992
(51) Int. Cl.: C12N 15/82, C12N 15/87

(54) **PARTICLE MEDIATED TRANSFORMATION OF COTTON**
PARTIKEL-AUSLÖSENDE TRANSFORMATION VON BAUMWOLLE
TRANSFORMATION DU COTON INDUITE PAR DES PARTICULES

(30) Priority: 06.03.1991 US 665374
(43) Date of publication of application: 17.03.1993
(73) Proprietor: MONSANTO COMPANY, St. Louis, Missouri 63167 (US)
(72) Inventor: McCABE, Dennis, E., Middletown, WI 53562 (US); MARTINELL, Brian, J., Madison, WI 53711 (US)
(74) Representative: von Menges, Albrecht
(86) International application number: US9201721
(87) International publication number: WO92015675

(56) References cited:
- EP-A- 0 301 749
- EP-A- 0 331 083
- WO-A-91/00359
- WO-A-91/00915
- TRENDS IN BIOTECHNOLOGY vol. 8, no. 6 , June 1990 pages 145 - 151 CHRISTOU, P., ET AL. 'Soybean genetic engineering - commercial production of transgenic plants'
- Bio/Technology, Volume 6, No. 8, issued August 1988, D.E. McCABE et al., "Stable Transformation of Soybean (Glycine max) by Particle Acceleration", pages 923-926, see pages 923-925.
- Plant Cell Reports, Volume 8, No. 10, issued 1990, J.J. FINER et al., "Transformation of Cotton (Gossypium hirsutum L.) via Particle Bombardment", pages 586-589, see page 586.
- In Vitro, Volume 26, No. 3, issued March 1990, J.J. FINER et al., "Structure of Introduced DNAs in Transgenic Cotton", page 70A, see entire document.
- P. AMMIRATO et al., "Handbook of Plant Cell Culture, Volume 3", published 1984 by MacMillan Publishing Co. (N.Y.), see pages 500-501, especially page 501.
- Indian Journal of Experimental Biology, Volume 24, No. 9, issued September 1986, Y.P.S. BAJAJ et al., "Micropropagation and Germplasm Preservation of Cotton (Gossypium spp.) through Shoot Tip and Meristem Culture", pages 581-583, see pages 581 and 583.

## Description

### Field of the Invention

The present invention relates to the field of genetic engineering in general and relates, in particular, to the genetic transformation of a major crop plant, i.e. cotton.

### Background of the Invention

It is a general object of modern biotechnology as applied to agriculture to be able to genetically engineer the major crop plants. Genetic engineering of crop plants is desired so that traits not normally present in the crop plants can be artificially introduced into the genome of elite lines of crop plants. Such artificial traits as insect resistance, herbicide resistance, or artificial manipulations of the agronomic qualities of the crop product, are possible once recombinant genes can be introduced into crop plants.

The first widely used technique to genetically engineer plants was based on the natural ability of the soil dwelling microorganism Agrobacterium tumefaciens. The Agrobacterium transformation technique was based on the natural ability of the bacteria to introduce a portion of its DNA into a plant cell as a part of its normal pathogenic process. By inserting the foreign trait which was sought to be introduced into the plant into the Agrobacterium in certain ways, the Agrobacterium could be used to transfer the genes into the plant. Agrobacterium transformation techniques have been developed for a number of plants, mostly of dicotyledonous plants, but has varied somewhat in its application from plant species to species.

It is a limitation of Agrobacterium-based mediation systems that they require cell culture or tissue culture of the plant tissues as a part of the process. The Agrobacterium transfers genes into one or more cells either in a suspension culture or in a callus culture and then the plants must be regenerated from such transformed cells in culture. For some species, such as cotton, Agrobacterium-mediated transformation is possible through a tissue culture technique such as this. However, there are some limitations on the application of this process. The first is that some plants, such as cotton, grow extremely slowly in tissue culture. Therefore a plant genetic engineering process utilizing Agrobacterium is an extremely long and often labor-intensive process. Also, tissue culturing methods for many plant species, again including cotton, do not work for all genotypes. Only certain cotton varieties are susceptible to widely practiced tissue culture techniques. The limitations of Agrobacterium transformation methods are further emphasized in cotton because the plants we call cotton are actually from more than one species. Some cultivars of cotton, such as Coker 312, and PD3 are cultivars of the species Gossypium hirsutum while the cultivars Pima (S6) and Sea Island (Barbados) are varieties of Gossypium barbadense. Therefore, while it is possible to genetically engineer a cotton plant of a cultivar which is adaptable to tissue culture, and then transfer the gene from that variety to other cotton varieties by traditional crop breeding techniques, it is a laborious process which can take multiple years to accomplish. Therefore, a plant genetic engineering method for cotton which would obviate tissue culture, and be genotype independent, would represent a significant advantage in speed and efficiency.

One technique which has been proposed for gene transfer into plants is based on a process of accelerated particle gene transformation. The first indication of the potential utility of this technique was a demonstration that genes could be coated onto tungsten particles and accelerated into onion skin, where the genes were transiently expressed, as is disclosed in U.S. Patent No. 4,945,050. Soon many researchers were conducting experiments in accelerated particle plant transformation to attempt to obtain transgenic plants.

A difficulty in the development of an accelerated particle transformation process is that the object is to obtain a germline plant transformation. By germline plant transformation it is meant that the germ cells of the plant are transformed in such a way that the progeny of the plant inherit the inserted gene. While it is readily possible to physically accelerate particles with genes on them into plant tissues, it is much more difficult to recover from those tissues a fully transgenic progeny plant which will pass the transgenic genes on to its progeny, what is referred to as a germline transformation event. This difficulty is compounded by the results obtained using most accelerated particle transformation techniques, in which the percentage of cells which are stably transformed by the particle acceleration process is a relatively small percentage of the cells of the treated tissue. This percentage can often be less than 1%, and sometimes an order of magnitude or two less than 1%. With the percentage of cells which are transformed being so small, the recovery of germline transformants can be difficult in view of the huge numbers of plants and tissues which have to be handled in order to find the few germline transformant events.

Nevertheless, in spite of these difficulties, plant genetic transformation has been achieved by accelerated particle plant transformation. One method of performing such a transformation is disclosed in European Patent No. 301,749 which disclosed the germline transformation of soybean plants and plant lines. One of the methodologies disclosed in that published patent application is based on accelerating particles coated with DNA into the excised embryonic axes of soybean plants. If such treated soybean embryonic axes are then treated with high cytokinin media, shoots can be induced to proliferate from the treated embryonic axes. If such shoots are cultivated into whole soybean plants, a significant percentage of the plants will have been found to have experienced germline transformation event.

In all prior art protocols based on accelerated particle genetic transformation, it has been a consideration in the development of the parameters of the accelerated particle process that damage to the tissues was to be limited to a minimum. For example, in the above mentioned U.S. Patent No. 4,945,050, it is specifically mentioned that the attempt of using the small particles is to achieve a minimum of cell disruption and lesions in the cell membrane. It has heretofore been considered an advantage of the accelerated particle method, particularly using very small carrier particles, in that damage to the treated tissues can be minimised so as not to disrupt growth structures of the tissues of the target organism.

The transformation of cotton via particle bombardment has been disclosed by Finer et al (Plant Cell Reports, 1990, vol 8, pp 568-589).

### Summary of the Invention

The present invention is summarised in that a method for the genetic engineering of cotton plants and lines by accelerated particle plant transformation is enabled which is independent of cotton genotype and which is not based on tissue culture or callus culture of the treated cotton tissues. The process is based on the insertion of DNA coated carrier particles into the excised embryonic axes from germinated cotton seed. The treated tissues are cultured in the medium with high levels of cytokinin content. A significant percentage of the shoots resulting from the procedure can be cultivated into complete clonal transgenic cotton plants without ever passing through a disorganized tissue culture (i.e. callus) stage.

The present invention thus provides a method of creating genetically transformed lines of cotton plants comprising the steps of:
(a) germinating cotton seed;
(b) isolating embryonic axes from the germinating cotton seed;
(c) placing the isolated embryonic axes on a target surface;
(d) preparing copies of a foreign genetic construction and coating the copies onto carrier particles which are small in size in relation to the size of the cells of cotton tissues;
(e) physically accelerating the carrier particles carrying the copies of the foreign genetic material at the target surface on which the embryonic axes are placed in such a fashion that many carrier particles lodge in the interior of cells of the embryonic axes;
(f) cultivating the embryonic axes as in step (e) on a medium substantially free from hormones and under conditions such that shoots arise from at least some of the treated embryonic axes without intervening callus culture; and
(g) cultivating those shoots that contain the foreign genetic construction in their genome into whole sexually mature cotton plants capable of transmitting the foreign gene by sexual inheritance to their progeny plants.

In a particular embodiment the method comprises the steps:
(a) germinating cotton seed;
(b) isolating embryonic axes from the germinating cotton seed;
(c) dissecting any embryonic leaves from the embryonic axes to expose the meristems of the axes;
(d) pre-treating the embryonic axes for particle treatment by culturing the embryonic axes in the dark on a medium with a cytokinin but substantially free of auxin;
(e) placing the isolated embryonic axes on a target surface,
(f) preparing copies of a foreign genetic construction including a foreign gene of interest and a marker gene;
(g) coating the copies of the foreign genetic construction onto carrier particles which are small in size in relation to the size of the cells of cotton tissue;
(h) physically accelerating the carrier particles carrying the copies of the foreign genetic material at the target surface on which the embryonic axes are placed in such a fashion that many carrier particles lodge in the interior of cells of the embryonic axes;
(i) cultivating the embryonic axes from step (h) on a medium substantially free from hormones and under conditions such that shoots arise from at least some of the treated embryonic axes without intervening callus culture;
(j) screening the shoots for expression of the marker gene; and
(k) cultivating those shoots that contain the foreign genetic construction in their genome into whole sexually mature cotton plants capable of transmitting the foreign gene by sexual inheritance to their progeny plants.

Alternatively the method may comprise the steps:
(a) germinating cotton seed;
(b) isolating embryonic axes from the germinating cotton seed;
(c) placing the isolated embryonic axes on a target surface;
(d) preparing copies of a foreign genetic construction including a marker gene and coating the copies onto carrier particles which are small in size in relation to the size of the cells of cotton tissues;
(e) physically accelerating the carrier particles carrying the copies of the foreign genetic material at the target surface in such a fashion that many of the carrier particles lodge in the interior of cells in the embryonic axes;
(f) cultivating the embryonic axes on a medium substantially free of hormones such that shoots with leaves arise from the treated embryonic axes without intervening callus culture;
(g) cutting a leaf from the plant and assaying a section of the leaf for expression of the marker gene;
(h) for shoots for which the assay revealed expression of the marker gene in the midrib of the leaf section, cultivating the shoot into a sexually mature plant;
(i) assaying the pollen of the plants for the expression of the marker gene to determine if the transformation involves a germ line event; and
(j) for those plants which involved a germ line event, recovering the progeny of the plants which contain the foreign genetic construction.

Other objects, advantages, and features of the present invention will become apparent from the following specification when taken in conjunction with the accompanying drawings.

### Brief Description of the Drawings

Fig. 1 is an exploded schematic view of a particle acceleration device for use within the process described within the present invention.
Fig. 2 is a top plan view of the apparatus of Fig. 1.
Fig. 3 is an illustration of the plasmid pTVBT41100 used in the examples below.
Fig. 4 is an illustration of the plasmid pCMC2114 used in the examples below.

### Description of the Preferred Embodiment

In accordance with the method of the present invention. a procedure is described for the genetic engineering of cotton plants which readily and repeatedly gives rise to transgenic cotton plants and lines without the need for tissue culture of cotton tissues. The procedure is designed to maximize the number of germline transformation events so that the transformation process can be carried out on a reasonable and practical scale while still resulting in large numbers of separately independently transformed cotton plants and lines. In contrast to other prior cotton transformation systems, this protocol has been found to be effective in a wide variety of cotton cultivars from different species of cotton. While other techniques rely on tissue culture generated plants which require obscure or outmoded cultivars which are particularly well suited for tissue culture, this technique has been found effective in current elite cotton lines.

This procedure is generally based on a process of propelling the carrier particles carrying the foreign gene into cotton embryos recovered from germinated cotton seeds. The embryos are conditioned for the particle acceleration process by treatment with a cytokinin but no auxin. The embryos are subjected to an accelerated particle "blast" that is more damaging to the tissues than those used in prior protocols. The resultant wounded cotton embryos can then be plated and cultured and will spontaneously give rise to shoots which develop into whole sexually mature plants. The percentage of transformant plants recovered seems to favorably compare with other transformation technologies in other plant systems.

The procedure described below is based on the use of dissected cotton embryos, derived from germinating seed. Generally, standard cotton seed can be surface sterilized and then placed under conditions for germination. The germinating seeds are isolated and from the seeds just germinated the cotton seed axis is removed and plated. The stage of germination of the embryo may be important. It has been found that using seed germinated to the point that one to four millimeters of the radicle is exposed has yielded good results. Neither the seed coat nor the cotyledons are used. It has been found that these germinating axes are particularly suitable for transformation and direct culture into plants without loss of tissue differentiation and without the need for callus culture of the cotton tissue. The embryos are then dissected to expose the meristem for blasting. This is done under microscope and involves removing the embryonic leaf or leaves obscuring the meristem. This is done to expose the meristem directly for the particle blast procedure

The explants thus produced are then pre-treated for the blast by culture in the dark on a culture medium with cytokinin but no auxin. This pre-treatment is believed helpful to transformation efficiency and to increase the number of germ line transformation events. The pre-treatment culture should extend from a few hours to a few days and, for cotton, include a cytokinin but no auxin. The culture should be in the substantial absence of light, i.e. either in the dark or in subdued light. For reasons that are not entirely clear, this pre-treatment protocol seems to increase the output of the procedure.

In general, in prior art procedures for the introduction of foreign genetic material into cells in general, and plant cells in particular, emphasis has been placed upon the minimization of any damages to the cells. It was thought that if large numbers of cells were disrupted or killed, that the tissues treated would be harmed so irreparably that it would be difficult to recover transformant tissues from the originally treated tissue. In past particle-mediated transformation processes, therefore, effort has been made to minimize the damage to the issues. This approach, however, also minimizes the number of transformed cells which are achieved, by ensuring that only a small fraction of the cells treated actually are injected with the carrier particle carrying foreign genetic material on them. Since the frequency at which target cells take up DNA from the carrier particle is small, if one is injecting only a percentage of the treated cells with carrier particles, an even smaller percentage of cells is actually transformed. Thus, the problem becomes to locate the very infrequent transformation events which occur from such a process.

By contrast, the method described herein is intended to maximize the number of cells into which a carrier particle is actually injected. Doing so carries a price, in that a large number of cells may be adversely affected by the procedure. In fact, many, if not most, of the cells treated by this procedure may be mortally wounded. It is believed that the process kills the majority of the cells in the embryonic meristem, leaving in the surviving axes a number of living cells barely sufficient to support the growth of a new shoot. Nevertheless, the procedure ensures that sufficient numbers of cells survive the procedure, with a carrier particle located in them, such that the frequency with which transformant events can be achieved is increased so that the overall procedure becomes practical. Since the object of the procedure is to recover a totally transgenic plant which is capable of passing the inserted gene on to its progeny, it does not matter if a large number of the treated cells die, as long as reliable numbers of cells can be treated which give rise to transgenic plants. Since the actual process of launching carrier particles at plant tissue is relatively economical and easy to perform, it becomes very practical to subject large numbers of plant tissues to an accelerated particle transformation procedure. Using the process described herein, transgenic plants of all lines of cotton can be recovered at a sufficiently high frequency that the overall process becomes very practical, and transgenic plants can be routinely and regularly recovered in significant numbers.

What has also been found by the applicants here is that in cotton the damaged cotton explants will remain in a viable state and these explants will give rise to growing viable cotton shoots at a reasonable frequency. While the reason that such shoots develop is not entirely clear, it may involve a process initiated as a wound response in cotton tissues following the disruptive particle blast. Whatever the cause for the phenomenon, shoots do arise and can quickly be cultivated into whole sexually mature cotton plants. While the shoots are plated on a medium containing cytokinin immediately after the blast, thereafter, hormone treatment is not necessary to foster shoot development. By suitable early screening of cotton shoots arising from blasted explants, the number of plants which must be cultivated into maturity can be limited to those likely to give rise to germline transformation events.

This process is based on the particle-mediated transformation of plant cells in general, and the cells of cotton in particular. The concept is that genetic material, either DNA or RNA, is coated onto small carrier particles. The carrier particles are then physically manipulated so that they are hurled at the cells of the cotton tissue. At some frequency the carrier particles lodge within the cytosol of the cotton plant cells. Also then at some frequency, and through a process not completely understood, the genetic material leaves the carrier particles and is integrated into the DNA of the host cotton cells. From those cells plants must be created either by regeneration or by normal cultivation of the plant cells. Along the way, either at the cellular or plant level, some combination of screening and selection must be employed to segregate the transformant plants from the nontransformant plants. In most particle-mediated plant transformation events, the nontransformant events will be the majority of the recovered plants. Therefore, it becomes necessary to provide a procedure by which sufficient numbers of plants can regularly be created that the relatively rare transgenic events can be readily and easily isolated. It is a desirable aspect of such a process that the percentage of transgenic plants recovered is the highest, since then the overall efficiency of the process is increased dramatically when less tissue area needed to be processed at the front-end to give the desired number of independent transformant events at the output end of the process.

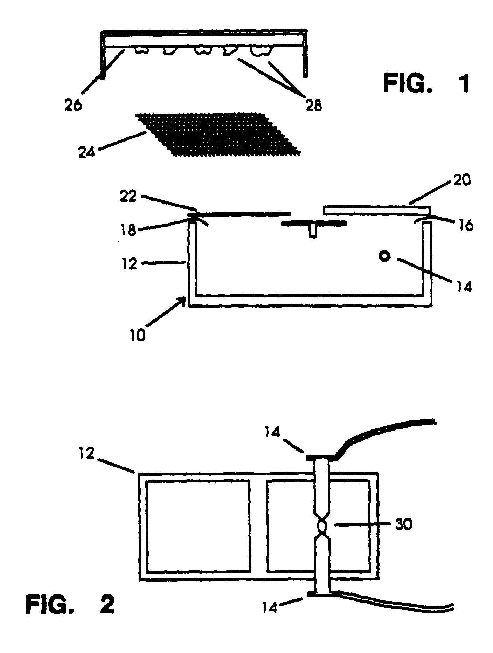

There are several factors which are to be considered in the creation of germ line plant transformation events. The genetic construction, be it DNA or RNA, must be constructed so as to be properly expressed in planttissues. The apparatus utilized in the process must be of a type capable of delivering the carrier particles with the coated genetic material on them into plant cells in such a fashion that a suitable number of cells can be transformed. There are many types of mechanical systems which can be envisioned to accelerate biologically inert small carrier particles into plant cells. Possible mechanisms include ballistic explosive acceleration of particles, centrifugal acceleration of particles, electrostatic acceleration of particles, or other analogous systems capable of providing momentum and velocity to small inert particles. A mechanism was used in the process of the present invention based on the acceleration of particles through an adjustable electric voltage spark discharge device which is capable of accelerating a planer carrier sheet at a target surface. Since this apparatus is particularly useful in the present invention, it will be described further below with reference to the accompanying drawing Figs. 1 and 2.

The particle acceleration apparatus is generally indicated at 10 of Fig. 1. The apparatus consists of the spark discharge chamber 12 into which are inserted two electrodes 14 which are spaced apart by a distance of approximately one to two millimeters. The spark discharged chamber 12 is a horizontally extended rectangle having two openings, 16 and 18, extending out its upward end. The opening 16 is covered by an access plate 20. The opening 18, located on the side of the rectangle of the spark discharge chamber opposite from the electrode 14, is ultimately intended to be covered by a carrier sheet 22. The electrodes 14 are connected to a suitable adjustable source of electric discharge voltage (not shown). A suitable source of electric discharge voltage includes a capacitor in the size range of one to two microfarad and the amount of the voltage of the charge introduced into the capacitor should be adjustable. Such an adjustable voltage can be introduced readily into such a capacitor through the use of an autotransformer which can be adjustable between a range of perhaps one and fifty thousand volts. It is preferred that suitable high voltage electric switching be provided so that the capacitor can safely be discharged through the electrodes 14 without harm to the human user of the system.

A carrier sheet 22 is intended to be placed upon the opening 18 of the spark discharge chamber 12. The carrier sheet 22 is a planer sheet of relatively stiff material which is capable of carrying small inert carrier particles thereon toward the target surface. It has been found that the carrier sheet 22 can preferably be a small sheet of aluminized saran coated mylar. Saran coated mylar is particularly light while having a high tensile strength. It is also envisioned that other light but relatively strong planar materials having some flexibility may also be used for the carrier sheet 22. The function of the carrier sheet 22 is to convert an outwardly outstanding force produced by the electrodes to a broadly distributed vertical force capable of accelerating a large number of carrier particles in parallel and with an even force. It can readily be understood that other kinds of force other than electric discharge can be used to impel the carrier sheet 22 upward. What is most particularly required is that the force be one which is adjustable so that the force of travel of the carrier sheet 22 when it is impacted can readily be adjusted.

Again referring to the apparatus of Figs. 1 and 2, above the opening 18 and the discharge chamber 12, in a position of approximately 15 millimeters above it, is a retaining screen 24. Placed above the retaining screen 24 at a distance of anywhere between 5 and 25 millimeters is a target surface 26. The target surface 26 can be any suitable culture surface onto which the material to be transformed can readily be placed. It has been found that an overturned petri dish can most conveniently be used for the transformation of plant tissues. Using a semisolid or solid agar-based medium in the bottom of a petri dish, it is possible to place tissues on the agar where they will readily be retained. Then placing the petri dish over the apparatus of Figs. 1 and 2, the petri dish itself can serve as the target surface while retaining the plant tissues on the agar placed therein.

Separately, copies of suitable genetic material, DNA or RNA, are separately prepared. These materials may be prepared by known techniques for the manipulation of DNA which are well known to those in the ordinary skill and art, and multiple copies of genetic constructions can readily be made. The genetic constructions must then be coated onto small carrier particles of a durable dense biologically inert material. Gold is a quite suitable and advantageous material for use as the carrier particles. The carrier particles must of extremely small size, typically in a range of one to three microns, so that they are very small in relation to the size of the target cells of the transformation process. It is preferred in particular that microcrystalline gold particles be used as carrier particles within the practice of the present invention. A suitable source of microcrystalline gold particles is a product referred to as "Gold powder A1570" from Engelhart Corporation of East Brunswick, New Jersey. This product consists of amorphous gold particles of high surface area and amorphous shape and size. In particular, it has been found that microcrystalline carrier particles of irregular size generally achieve a transformation efficiency which is higher than that which can be achieved by spherical gold particles, for reasons that are not well understood. In at least one set of experiments, a mixture of the microcrystalline gold powder and gold spheres, 1-3 micron in size, was used with particularly good success as well.

The genetic material to be inserted into the cells is then coated onto the carrier particles. This can be readily done by precipitating solutions of DNA or RNA onto the carrier particles themselves. Suitable stabilizers can be added to the mixture to help with the longevity of the genetic material on the carrier particles, such as the preparation based on spermidine described in the examples below. The carrier particles with the genetic construction placed thereon are then layered onto the top of the carrier sheet 22. The layering is done so as to distribute a relatively even pattern of carrier particles across the entire top surface of the carrier sheet 22. Within the present process, the applicants have applied the coated carrier particles to the carrier sheet at a loading rate in the range of .0125 to .050 milligrams of coated carrier particles per square centimeter of carrier sheet. The carrier sheet 22 is then placed upon the proper opening on the top of the discharge chamber 12 which is the opening 18. The target surface 26, including the living plant material thereon, is then placed in position above the retaining screen 24. A small droplet of water, preferably of about 10 microliters in size, is then placed in the chamber bridging between the ends of the two electrodes 14. The access cover 20 is placed in position on top of the spark discharge chamber 12.

At this point, the entire apparatus is enclosed in a vacuum chamber and a vacuum is drawn down into the range of approximately 500 millimeters of mercury. As the vacuum is being drawn, a supply of helium is bled into the vacuum chamber to thus replace the remaining atmosphere within the chamber with helium. Thus the vacuum chamber contains within it a relative vacuum compared to the atmosphere and the air within the vacuum is disproportionately displaced with helium. The lower density of helium, combined with the reduced pressure, helps to lower the drag both on the carrier sheet 22 and on the carrier particles themselves.

At this point the accelerated particle transformation process can be initiated. This is done by electrically discharging the voltage from the capacitors to the electrodes 14. The voltages used in the present process have usually been in the range of 10-25kV and the range of 16-22kV is now preferred. The voltage discharge is preferably effectuated through the use of appropriate electric switching described above. The force of the electric discharge initiates a spark which leaps the gap between the electrodes 14 instantly vaporizing the small droplet of water which was previously placed therebetween. The force of the vaporization of that water creates a violent atmospheric shock wave within the air placed inside of the spark discharge chamber 12. The shock wave radiates outward from the electrodes in all directions. The radiating shock wave impacts the carrier sheet 22 which is then propelled upward with great velocity. The upwardly traveling carrier sheet 22 accelerates upward at great force until it contacts the retaining screen 24. It is at this point that the displacement of the remaining atmosphere in the chamber with helium assists in the travel of the carrier sheet 22, since helium provides the less drag on the flight of the carrier sheet as well as on the carrier particles themselves. At the retaining screen 24, the carrier sheet 22 impacts the retaining screen 24 and is totally retained thereon. The carrier particles previously coated with the genetic material, by contrast, fly off of the carrier sheet and travel freely onward toward the target tissues. The small carrier particles then proceed to impact the living tissue on the target surface and proceed into the cells of the tissues placed thereon.

As may be readily seen by a review of the apparatus in this process, the actual momentum and velocity of the carrier particles as they impact the surface of the target tissue is adjustable by adjusting the voltage of the initial electric discharge applied to the electrodes 14. Thus, by varying the amount of the electric discharge applied across the electrodes 14, the velocity by which the particles impact the target can be adjusted, and thus the depth of penetration of the carrier particles into the tissue of the target tissues can be adjusted continuously throughout the range of adjustment provided for by the electric voltage applied across the electrodes 14. The actual critical physical step is the acceleration of the carrier sheet 22 toward the target surface. The provision for layering the carrier particles onto the carrier sheet 22, and accelerating the carrier sheet 22 toward the target, ensures that a broad horizontal layer of carrier particles will travel toward the target tissues. In this way, a relatively large number of transformant events can be conducted at one time since a relatively broad horizontal range of area on the target surface 26 is impacted by an array of carrier particles traveling at similar velocities. Other mechanical mode of means could be used, other than spark discharge, to provide the mode of force for the carrier sheet 22 as long as they were suitably adjustable.

To be useful in a particle-mediated transformation process, the transforming exogenous genetic construction must be capable of performing some useful function in the cells of target plant tissues. The transforming genetic construction, be it DNA or RNA, will normally be a chimeric construction in the sense that its genetic material originates from more than one kind of organism. The genetic construction would be one that is capable of expressing in the target tissues a gene product of use. Such gene products will typically be a foreign protein which it is desired to express in the cells of the plant tissue but could be other gene products as well, as such as an antisense RNA construct intended to inhibit an endogenous plant system. Typical foreign genetic constructions are intended to the expression of proteins implant cells and often usefully embodied in expression cassette vectors for plant cells, many of which are known to those of ordinary skill in the art. Typically such a plant expression vector system includes, besides the coding sequence for the desired exogenous or foreign gene, appropriate flanking regulatory sequences suitable for the expressing of the foreign gene in the plant cells. The appropriate flanking regulatory sequences would include a promoter sequence capable of initiating transcription and a translational terminator to terminate translation of an RNA message if protein synthesis is desired. It has also previously been demonstrated that typical promoters and transcription terminators found to be effective in other plant tissues are effective in cotton as well. It may also be desirable to incorporate between the promoter and the coding region of the genetic sequence a translation or transcriptional enhancer to aid in the efficacy of the inserted genetic material into the transgenic plants.

It is desirable if the transforming genetic construct includes a marker gene which is capable either of selection or screening in the treated plant tissues. Selectable markers are generally preferred for plant transformation events, but are not available for all plant species. A selectable marker is one that conditions for a trait in the transformed plant cells which can be selected by the exposure of the plant tissues to a selection agent. Suitable selectable markers would be antibiotic resistant genes or herbicide resistant genes which, when inserted in some cells of a plant in culture, would imbue those particular cells with the ability to withstand exposure to the antibiotic or the herbicide which would kill all the nontransformant cells in the culture. Another type of marker gene is one that can be screened by ready histochemical or biochemical assay even though the gene cannot be selected for. In this instance it is most desirable if the gene codes for a phenotypic trait which can readily be observed in transgenic plants. A suitable marker gene which has been found useful in such plant transformation experience is the Gus gene as described by Jefferson, et al. EMBO J., 6:3901-3907 (1987 ). The Gus gene, coding for the enzyme beta-glucuronidase, which can be expressed in plant cells and the expression of the Gus gene can be tested, in a tissue destructive assay, for its presence in plant tissues. The Gus gene will turn a convenient substrate, indigo-glucuronide, or 5-bromo-4-chloro-3-indolyl glucuronide blue in color in an in situ assay in plant tissues. Thus, the use of a Gus gene provides a convenient colorimetric assay for the expression of introduced DNA in plant tissues by phenotypic analysis in the plant tissues. Thus, in a typical transformation process, the desired gene of interest sought to be expressed in the plant would be coupled in tandem in a single genetic construct on a single DNA strand with the Gus gene. Then the coupled tandem genetic construct would be transformed into plant tissues and the resultant plant tissues would be analyzed for expression of the Gus enzyme in the target plant tissues to find transgenic tissues from the treated tissues.

In the instance of the Examples described below, no selectable marker was used. Instead the Gus gene marker was used as a screening marker. Since the applicants recover transgenic shoots at a reasonably high frequency (approx. 1%), selection is not necessary for the process to be efficiently and economically performed. The use of a selectable marker would add to the efficiency of the system, if one were available.

In the present process, as performed in cotton, the treated embryonic axes which survive the blasting protocol each give rise to a single shoot. In a transformation procedure for soybean, practiced by the inventors here and others, multiple shoots were found to arise from blasted embryonic axes, and some of the shoots were found to be clonal transformants, suggesting that the shoots arose from a single cell source. In cotton, the phenomenon which is responsible for achieving germ line transformation success appears to be different. A single shoot arises from each treated cotton embryonic axis, and the shoot is typically chimeric, or only partially transformed. However, the transformed portion of the cotton plant can be "enriched" by testing the leaves of the growing plant for activity of the marker gene (Gus), and by selectively pruning the growing plant to favor the growth of the expressing portion of the plant. In cotton, this technique has proven successful in generating plants with a sufficiently transformed character that germ line transformation is achieved. The germ line character of the transformed RO plant (first transgenic plant) can be assessed by gus screen of the pollen it sheds. Not only does the gus screen work effectively with cotton pollen grains, the segregation pattern of the transgenic gene can be assessed by the relative numbers of expressing and non-expressing pollen grains. For a single insert of the foreign gene, Mendelian inheritance will dictate that 50% of the pollen grains will carry the transgenic gene insert. The confirmation of the germ line nature of the transformation is confirmed by expression of the gene in progeny (R1) plants, which will pass the insert gene on to their progeny through normal patterns of genetic inheritance.

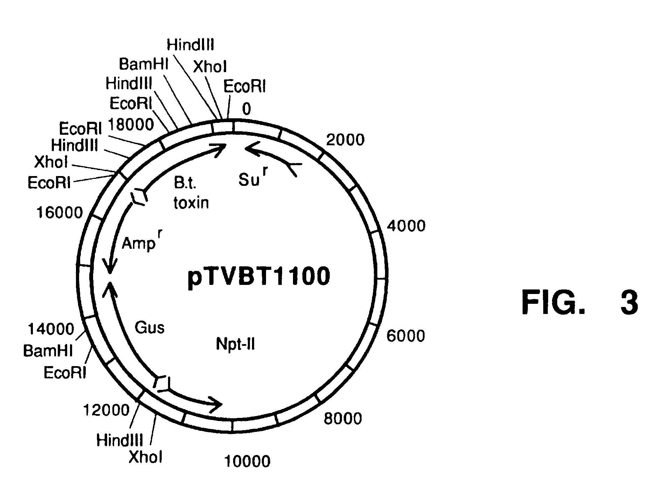

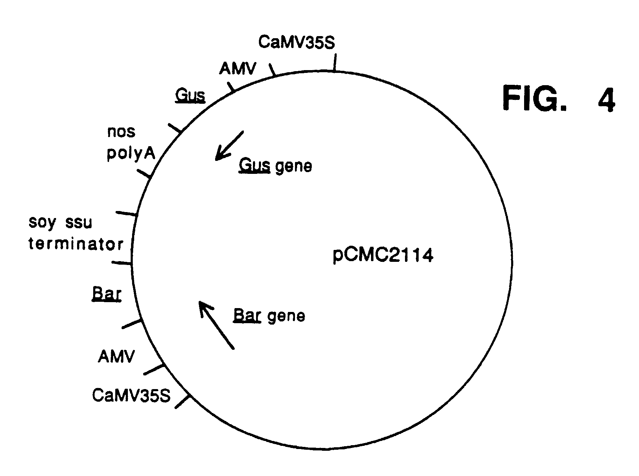

In the Examples below, two different plasmid vectors are used. One vector is pTVBT41100, illustrated in Fig. 3. This vector includes a gene coding for NPT-II, which provides resistance to kanamycin, an expression cassette for the Gus gene, two antibiotic resistance markers useful in bacterial hosts (ampicillin resistance, Amp^{r} and sulfadiazene Su^{r}), and an expression cassette for the Bacillus thuringiensis (B.t.) insect specific toxin. The B.t. expression cassette includes an untranslated 5' leader sequence from alfalfa mosaic virus (AMV), the 35S promoter from cauliflower mosaic virus (CaMV35S) and a synthetic coding sequence derived from plant codon preferences which codes for the amino-terminal 55% of the Lepidopteran specific B.t. toxin from B.t. variety kurstaki. The plasmid pCMC2114, illustrated in Fig. 4, contains two oppositely oriented gene constructs, one being a Gus gene construct with the CaMV35S promoter, an AMV 5' leader and the nopaline synthase polyadenylation region, and the other being a Bar gene from Streptomyces hygroscopius with the CaMV35S promoter, an AMV 5' leader and the soybean SSU terminator. The Bar gene codes for the enzyme phosphinothricin acetyltransferase which provides resistance to glutamine synthase inhibitor herbicides. The plasmid pCMC2114 is available from the ATCC, Accession No. 67,936. The Gus gene can readily be isolated from copies of pCMC2114 by digestion of copies of the plasmid with Hind III and Sal I, with the Gus gene cassette located between the sites for these two restriction enzymes. It is to be understood, however, that neither plasmid is essential to the practice of the present invention, but are instead exemplary. While the Gus gene is a convenient and useful screening marker for this process other screening techniques, including molecular screening techniques such as polymerase chain reaction (PCR) for the inserted gene of interest are also possible.

### Example 1

### Surface Sterilization

The process began with commercial cotton seed. Seeds of Pima cotton (variety S6) were used. The process begins with sieve beaker system which may be autoclaved. This is a beaker with dozens of holes drilled in its bottom that can be nested inside a non-drilled glass beaker. It is also useful to utilize a third sterile beaker for rinsing the seeds so that the sieve beaker can be rested in the sterile beaker while discarding wash water.

The sieve beaker was filled with cotton seeds. The beaker into which the sieve beaker is nested was then filled with 95% ethanol until the seeds were covered. The seeds were then drained. Following that, the seeds were rinsed with distilled water three times, after which they were drained again. Alternatively, some of the seed was covered with bleach without prior treatment. At this point, the seeds were exposed to a mixture of 50% clorox bleach so as to cover the seeds and the seeds were allowed to rest within the bleach solution for three minutes. The bleach was drained and the seeds were then rinsed five times with distilled water.

The surface sterilized cotton seeds were then placed in a sterile glass beaker, to which was added a cotton antibiotic sterilization medium consisting of sterile distilled water to which has been added carbenicillin at 200 mg per liter, cefotaxime at 125 mg per liter, and 30 mg each of Bravo WP, Benlate 50 DF, and Captan 50 WP, per liter. Twice as much of the cotton antibiotic sterilization medium was used by volume as there were seeds. The seeds were incubated in the sterilization medium for three to four hours and in the dark at room temperature. The process was begun at 8:00 a.m., the seeds were incubated until about noon.

Then the seeds were drained by pipette, rinsed with sterile distilled water, and the beaker refilled with fresh cotton antibiotic sterilization medium followed by an additional three hours of incubation.

The seeds were then drained and the seeds were plated on the surface of a 0.8% water agar plus ccb, a mixture of the antibiotics and fungicides carbenicillin (200 mg/l), Cefotaxime (50 mg/l), and Benomyl (60 mg/I). All ungerminated seeds and seed coat debris was removed. The seeds were quite congested on the agar plate but remained in a single layer. The seeds were then incubated overnight at 15° C in the dark so as to germinate. If germination proceeded appropriately, the seed germination could be stopped by refrigeration at 4° C, for up to three days following the germination process. Alternatively, the seeds, while still covered with the cotton antibiotic sterilization medium, were incubated at 28° C overnight, then drained and transferred in bulk to a 15° C incubator for immediate use.

### Seed Dissection

After the germination of the seeds, or the removal of the germinated seeds from storage, seeds were selected that were just beginning to germinate. Overly germinated or ungerminated seeds were discarded. The proper stage of germination was defined as fully imbibed seed with one to four millimeters of the radicle exposed. Under sterile conditions, the seed axis was removed out of the seed. This was done by manual manipulation with gloved hands to remove the seed axis from both of its cotyledons and its seed coat. The process was relatively easy to perform with practice. It is possible to readily develop the ability to pop the seed coat axis apart from the seed, without damaging the seed axis, or leaving any of the cotyledon on the seed axis.

The excised seed axis was then washed in three to four rinses of sterile distilled water. The washed but undissected explants were either dissected immediately or were stored by plating on standard OR ccb medium made with fresh benzylaminopurine or BAP, but no NAA. This media was that described by Barwhale et al., Planta, 167, pp. 473-481 (1986), but without the NAA hormone. The explants were plated on the agar surface by being laid on their side. The excised embryonic seed axes plated on the agar medium were incubated at 4°C in the dark for up to three days.

### Exposing the Meristem

The washed seed axis explants were now ready for micro dissection to expose the meristems of the seed axes. This dissection was performed under sterile distilled water and with sterile tools. The dissection consisted of removing the embryonic leaf, or leaves if there was more than one, that obscures the meristem on each of the excised seed axes. The fully dissected explants were transferred to another petri dish containing sterile distilled water.

### Pre-Blast Hormone Treatment

After all the dissections were completed, the explants were again washed in three to five rinses of sterile distilled water. The free water was removed by pipette after the final rinse. The treated explants were then again laid on their side on the surface of standard OR ccb medium again made with fresh BAP but no NAA. The explants were incubated overnight, 24 hours maximum, at 15° C in the dark. The treated excised embryonic axes with exposed meristems are now ready for the accelerated particle transformation blast.

### Genetic Material and Carrier Particle Preparation

Ten milligrams of amorphous crystalline gold powder, or of an equal mixture of 1-3 micron gold spheres and crystalline gold powder, was measured into the bottom of a 1.5 milliliter Eppendorf microfuge tube. Care was taken to ensure that the gold did not spill on the sides of the tube, since that would make it difficult to resuspend the gold due to the small volumes used in the preparation process. To this microfuge tube was added 100 microliters of 0.1 M spermidine (free base) and the microfuge tube was vortexed well. To the microfuge tube was then added 1 to 10.0 micrograms of double-stranded DNA of the foreign genetic material and the tube was then vortexed gently but completely The DNA utilized was of pAMVBT41100. While the DNA/carrier particle mixture was gently vortexed, 100 microliters of 2.5 M CaCl₂ was added to the tube. Then the vortex was ceased, and precipitation was permitted for 10 minutes at room temperature. The preparation could be stored at this point for some time. Shortly before use, the mixture of DNA and carrier particles was given a brief spin in a microfuge. The cleared supernatant was removed completely, and the precipitant consisting of the DNA and carrier particles was resuspended in 20 milliliters of 100% ethanol. The resuspended DNA and carrier particle mixture was then sonicated in a water bath sonicator for two to three brief one second exposures. The resulting suspension was then coated onto the carrier sheet 22 at a rate of 320 microliters per carrier sheet, or a calculated rate of 0.05 milligrams per square centimeter of the carrier sheet. After allowing the gold to settle, the excess ethanol was drained away and the sheet dried. These preparations of DNA and carrier beads were made fresh daily.

### Blasting.

At this point in the process, the carrier sheets were placed upon the apparatus of Figs. 1 and 2 for the blasting process. The cotton explants were plated on 12% xanthan gum target plates. Using the normal germination and pre-blast hormone treatments described above, typically 25 explants were found to fit on each of the target surface within the blast area.

The parameters used for the particle-mediated transformation blast itself included a relatively high electric discharge voltage through the gun, typically in the range of 15-25 kilovolts. The standard voltage used was 18 kv. The voltage was adjusted to achieve a level of impact on the treated axes such that the rate of survival of the meristems was between 40% and 80%. In other words, the blast force was adjusted to a level such that at least some of the meristems were rendered non-viable by the process. The blasting experiments were conducted at 350 millimeters of mercury, with helium introduced at a rate of 1.5 liters per minute at atmospheric levels, and approximately 5.0 liters per minutes under the vacuum.

Each of the target tissues was blasted twice during the same day, once in the morning and once in the afternoon, with the explants stored between successive blasting procedures in a moist chamber at approximately 28° C in the dark and with low oxygen (8%). The target tissues were placed in the dark immediately after each blasting exposure.

### Post-Blast Protocol

The explants were now removed from the target surface, and plated in a normal orientation on OR ccb medium made with fresh BAP but no NAA. Care was taken not to expose the explants to excessive light. Care was exercised not to allow the meristem to come in contact with any media, and no wet plates were utilized. The fresh explants were plated and then incubated at 28° C in the dark and with low oxygen for two full days.

One day after the blasting, a preliminary assessment of transient enzyme activity was conducted on the resultant tissues. The assay was conducted at this time to check for the quality of the bead preparation protocol, and also to look specifically at the number of transformation events in the meristem, a rough approximation of which can be made by checking the transient activity of the explants at this stage. Although due to the heavy damage from the blasting process, 20 to 60% of the meristems were sufficiently damaged so as to never produce shoot, those same damaged meristems will, upon assay, exhibit excellent transient gene activity particularly of the Gus gene using this procedure. Thus, the tissues can be assayed at this step for the percentage of Gus activity, even though shoots are not yet evident on the meristems subjected to the procedure.

Following the initial post-blast incubation on the medium described above, the cotton explants were transferred to dextrose-based woody plant medium (WPM), minus BAP plus carbenicillin and benomyl, in plantcons again under low light. The WPM medium mixture, based on McCown & Lloyd, Proc. International Plant Propagation Soc., 30:421 (1981), was prepared as follows: NH₄NO₃ (400 mg/l), Ca(NO₃)₂•4HOH (556 mg/l), K₂SO₄ (990 mg/l), CaCl₂•2HOH (96 mg/l), KH₂PO₄ (170 mg/l), H₃BO₃ (6.2 mg/l), Na₂MoO₄•2HOH (0.25 mg/l), MgSO₄•7HOH (370 mg/l), MnSO₄•HOH (16.9 mg/l), ZnSO₄•7HOH (8.6 mg/l), CuSO₄•5HOH (0.025 mg/l), FeSO₄•7HOH (27.8 mg/l), Na₂•EDTA (37.3 mg/l), Thiamine•HCl (1.0 mg/l), Nicotinic acid (0.5 mg/l), Pyridoxine•HCl (0.5 mg/l), Glycine (2.0 mg/l), Myo-inositol (100 mg/l), Dextrose (20 g/l), Agar (3.0 g/l), Gelrite (1.1 g/l), Calcium gluconate (1.29 g/l), Carbenicillin (200 mg/l) and Benomyl (60 mg/l). The tissues were incubated at 28° C in the dark with normal oxygen for five to seven days.

Following the above culturing in the dark, the plantcons were then moved to a shade regimen. The explants were left in the shade for two full days to become accustomed to gradual light exposure.

Following that the plantcons containing the same media were then transferred to full light exposure so as to induce shoot development in the tissues under cultivation.

### Identification of Transformant Events

The plantcons were thus moved to a cultivation chamber and exposed to 16 hour light periods at 28° C. A number of cultured explants then would proceed to exhibit shoot elongation and development from the plated tissues. It then became necessary to evaluate the growing shoots to ascertain the level of germ line transformation events which were achieved through this process. The assay procedure was conducted at such a point that the shoots each had developed their first leaves. Then the outermost one-third to one-half of each leaf was cut off completely across the leaf through the midrib. The leaves were then assayed for Gus activity to identify Gus-positive expressing plants.

At this point, the quality of the event was characterized depending on the level of Gus activity in the leaf. Some of the leaves exhibited only uneven or irregular Gus expression indicating chimeric plants. Based on the results below, and on experience with other plant systems, it has been observed and verified that for chimeric plants, a transformation of the vascular system, as exemplified by the leaf midrib, correlates very well with the occurrence of a germ line transformation event. Some of the leaves seemed to be totally blue, indicating putatively clonal transgenic plants. If the plant was characterized as germ line, the plant was transferred into rooting conditions and grown out in the greenhouse. For chimeric plants, the plant was pruned to just above the transformed leaf so as to force the axillary bud to grow from the transformed area of the plant after it was retested.

For plants that tested negative, the leaves were removed, and the plants were cultured until newly formed leaves were regenerated. Tests were again conducted. This process was repeated three times before a final negative determination for the plants was made.

In the experiment as described above, 590 shoots of pima cotton were recovered. From those shoots, six shoots were determined to exhibit significant Gus activity throughout the plant. No grafting of the transformant shoots was needed, and root elongation and development occurred normally. From the 590 shoots, six putatively transgenic plants had been recovered.

The plant that has grown the farthest has been verified to have complete clonal transformation and to be able to pass its inserted gene on through Mendelian inheritance to its progeny. The plant is totally clonal, exhibiting Gus activity in all its leaves, and in all sections of the plant. The pollen of the plant was tested for Gus activity, and approximately half the pollen grains of the plant were found to be expressing Gus, at the rate that would be expected given normal segregation of genes in germ cells. Self pollination of the clonal RO plant produced progeny plants which contain and express the inherited gene inserted in the transgenic parent plant. The segregation of the inserted gene among the progeny plants was about three to one, or what would be expected for a heterozygous allele. Accordingly, Mendelian inheritance of the gene has been confirmed and a germ line transformation of Pima cotton has thus been confirmed. In addition, two other plants exhibited transformation, so far, including the shed of expressing pollen segregating at about one to one.

The entire process as described above, from initial plating of the seeds to the recovery of an initial generation, i.e. R-0, transgenic plant requires approximately three to five weeks. Based on the initial results as described above, it is expected that approximately one clonal transgenic plant will occur per approximately 100 to 500 meristems exposed to the blasting process. Of the shoots produced from the process, approximately. 1-1 % will be found to have experienced germ line transformation events.

### Example 2

The above protocol is in the process of being repeated with tissues of cotton varieties Delta & Pine 50, an upland cotton, Delta & Pine 90, which is an Acala variety, and Sea Island, variety Barbados. The plasmid being used is pCMC2114. Shoots have been recovered, initially assayed and are in cultivation to yield plants. To date, leaf cutting assays indicate transformation levels consistent with the results from Pima above. In addition, the assays have revealed transformation events which resulted in transformation of vascular tissues, which are associated with germ line transformation events.

In this way, it now becomes both feasible and practical to obtain transgenic cotton plants of a wide variety of genotypes and varieties. While the process described above does involve the use of tissue culture apparatus, such as petri plates and plant growth media, it does not require tissue culture in the sense that there is no undifferentiated plant tissue at any step in the process. Since cotton is a notoriously slow growing system in undifferentiated plant culture, the fact that no undifferentiated tissue is created through this process is a unique advantage of this procedure and allows for the ready and convenient, and rapid, generation of large numbers of transgenic cotton plants with a minimum of difficulty and expense.

## Claims

1. A method of creating genetically transformed lines of cotton plants comprising the steps of:
(a) germinating cotton seed;
(b) isolating embryonic axes from the germinating cotton seed;
(c) placing the isolated embryonic axes on a target surface;
(d) preparing copies of a foreign genetic construction and coating the copies onto carrier particles which are small in size in relation to the size of the cells of cotton tissues;
(e) physically accelerating the carrier particles carrying the copies of the foreign genetic material at the target surface on which the embryonic axes are placed in such a fashion that many carrier particles lodge in the interior of cells of the embryonic axes;
(f) cultivating the embryonic axes as in step (e) on a medium containing a cytokinin immediately after the blast and on a medium substantially free from hormones and under conditions such that shoots arise from at least some of the treated embryonic axes without intervening callus culture; and
(g) cultivating those shoots that contain the foreign genetic construction in their genome into whole sexually mature cotton plants capable of transmitting the foreign gene by sexual inheritance to their progeny plants.

2. A method according to claim 1, wherein in step (a), the seed is germinated until one to four millimetres of the radicle is exposed.

3. A method according to claim 1 or 2, wherein after the embryonic axes are isolated from the seed, the embryonic axes are dissected to remove any embryonic leaves from covering the meristems.

4. A method according to claim 1,2 or 3 wherein after step (b), the embryonic axes are pretreated by culture in the substantial absence of light in a medium having a cytokinin but no auxin.

5. A method according to any one of the preceding claims where after step (e), the embryonic axes are incubated on a medium having a cytokinin but no auxin in the substantial absence of light for a brief time.

6. A method according to any one of the preceding claims wherein in step (d) the copies of the foreign genetic construction includes both a foreign gene of interest and a marker gene, and wherein the shoots from step (f) are screened for the expression of the marker gene to identify shoots carrying the foreign genetic construction.

7. A method according to claim 6 wherein the marker gene is the Gus gene.

8. A method according to any one of the preceding claims wherein in step (e), the physically accelerating step is performed with sufficient force and intensity that between 20 and 60 percent of the embryonic axes are so damaged that they never give rise to a shoot in step (f).

9. A method according to any one of the preceding claims wherein in step (e), the physically accelerating step is performed with sufficient force and intensity that the majority of cells in the embryonic meristems are killed.

10. A method according to any one of the preceding claims wherein step (e) is performed by placing the coated carrier particles on a planar carrier sheet, accelerating the carrier sheet at the target surface with a shock wave of adjustable force, and restraining the carrier sheet in such a manner that the carrier particles may travel on toward the target surface.

11. A method according to claim 10 wherein the shock wave of adjustable force is created by an electric spark discharge and the force is adjustable by adjusting the electric voltage of the discharge.

12. A method according to any one of the preceding claims wherein the cotton seed is of a variety selected from the group consisting of Pima, Avala and Upland Cotton varieties.

13. A method of creating genetically transformed lines of cotton plants comprising the steps of:
(a) germinating cotton seed;
(b) isolating embryonic axes from the germinating cotton seed;
(c) dissecting any embryonic leaves from the embryonic axes to expose the meristems of the axes;
(d) pre-treating the embryonic axes for particle treatment by culturing the embryonic axes in the dark on a medium with a cytokinin but substantially free of auxin;
(e) placing the isolated embryonic axes on a target surface;
(f) preparing copies of a foreign genetic construction including a foreign gene of interest and a marker gene;
(g) coating the copies of the foreign genetic construction onto carrier particles which are small in size in relation to the size of the cells of cotton tissue;
(h) physically accelerating the carrier particles carrying the copies of the foreign genetic material at the target surface on which the embryonic axes are placed in such a fashion that many carrier particles lodge in the interior of cells of the embryonic axes;
(i) cultivating the embryonic axes from step (h) on a medium containing a cytokinin immediately after the blast and on a medium substantially free from hormones and under conditions such that shoots arise from at least some of the treated embryonic axes without intervening callus culture;
(j) screening the shoots for expression of the marker gene; and
(k) cultivating those shoots that contain the foreign genetic construction in their genome into whole sexually mature cotton plants capable of transmitting the foreign gene by sexual inheritance to their progeny plants.

14. A method according to claim 13 wherein the marker gene is the Gus gene.

15. A method according to claim 13 or 14, wherein in step (h), the physically accelerating step is performed with sufficient force and intensity that between 20 and 60 percent of the embryonic axes are so damaged that they never give rise to a shoot in step (i).

16. A method according to claim 13, 14 or 15 wherein in step (h), the physically accelerating step is performed with sufficient force and intensity such that the majority of cells in the meristem of each axis are killed.

17. A method according to any one of claims 13 to 16 wherein step (h) is performed by placing the coated carrier particles on a planar carrier sheet, accelerating the carrier sheet at the target surface with a shock wave of adjustable force, and restraining the carrier sheet in such a manner that the carrier particles may travel on toward the target surface.

18. A method according to claim 17 wherein the shock wave of adjustable force is created by an electric spark discharge and the force is adjustable by adjusting the electric voltage of the discharge.

19. A method according to any one of claims 13 to 18 wherein the cotton seed is a variety selected from the group consisting of Pima, Acala and Upland Cotton varieties.

20. A method of creating genetically transformed lines of cotton plants comprising the steps of:
(a) germinating cotton seed;
(b) isolating embryonic axes from the germinating cotton seed;
(c) placing the isolated embryonic axes on a target surface;
(d) preparing copies of a foreign genetic construction including a marker gene and coating the copies onto carrier particles which are small in size in relation to the size of the cells of cotton tissues;
(e) physically accelerating the carrier particles carrying the copies of the foreign genetic material at the target surface in such a fashion that many of the carrier particles lodge in the interior of cells in the embryonic axes;
(f) cultivating the embryonic axes on a medium containing a cytokinin immediately after the blast and on a medium substantially free of hormones such that shoots with leaves arise from the treated embryonic axes without intervening callus culture;
(g) cutting a leaf from the plant and assaying a section of the leaf for expression of the marker gene;
(h) for shoots for which the assay revealed expression of the marker gene in the midrib of the leaf section, cultivating the shoot into a sexually mature plant;
(i) assaying the pollen of the plants for the expression of the marker gene to determine if the transformation involves a germ line event; and
(j) for those plants which involved a germ line event, recovering the progeny of the plants which contain the foreign genetic construction.

21. A method according to claim 20 wherein the marker gene is the Gus gene.

## Patentansprüche

1. Verfahren zur Erzeugung genetisch transformierter Linien von Baumwollpflanzen, bei dem man
(a) Baumwollsamen keimen läßt,
(b) von den keimenden Baumwollsamen embryonische Achsen isoliert,
(c) die isolierten embryonischen Achsen auf einer Zieloberfläche plaziert,
(d) Kopien einer fremden genetischen Konstruktion präpariert und die Kopien auf Trägerpartikeln beschichtet, die im Verhältnis zu der Größe der Zellen des Baumwollgewebes klein sind,
(e) die Trägerpartikel, die die Kopien des fremden genetischen Materials tragen, an der Zieloberfläche, auf der die embryonischen Achsen plaziert sind, derart physikalisch beschleunigt, daß viele Trägerpartikel in das Innere der Zellen der embryonischen Achsen hineingeschossen werden,
(f) die embryonischen Achsen wie in Stufe (e) auf einem im wesentlichen Hormon-freien Medium und unter Bedingungen kultiviert, so daß aus wenigstens einigen der behandelten embryonischen Achsen Triebe entstehen, ohne die Kalluskultur zu beschädigen, und man
(g) diese Triebe, die die fremde genetische Konstruktion in ihrem Genom enthalten zu vollständigen, sexuell ausgereiften Baumwollpflanzen kultiviert, die in der Lage sind, das Fremdgen durch sexuelle Vererbung auf ihre Nachfolgepflanzen zu übertragen.

2. Verfahren nach Anspruch 1, bei dem man in Stufe (a) den Samen keimen läßt, bis 1 bis 4 mm der Wurzel hervorgetreten sind.

3. Verfahren nach Anspruch 1 oder 2, bei dem man die embryonischen Achsen, nachdem man sie von den Samen isoliert hat, zerlegt, um alle embryonischen Blätter zu entfernen, die die Meristeme bedecken.

4. Verfahren nach Anspruch 1, 2 oder 3, bei dem man die embryonischen Achsen nach Stufe (b) durch Kultivieren im wesentlichen unter Ausschluß von Licht in einem Medium vorbehandelt, das ein Cytokinin, aber kein Auxin enthält.

5. Verfahren nach einem irgendeinem der vorangegangenen Ansprüche, bei dem man nach Stufe (e) die embryonischen Achsen im wesentlichen unter Ausschluß von Licht für eine kurze Zeit auf einem Medium inkubiert, das ein Cytokinin, aber kein Auxin enthält.

6. Verfahren nach irgendeinem der vorangegangenen Ansprüche, bei dem in Stufe (d) die Kopien der fremden genetischen Konstruktion sowohl ein interessierendes Fremdgen als auch ein Markergen einschließt und bei dem man die Triebe aus Stufe (f) daraufhin screent, ob sie das Markergen exprimieren, um Triebe zu identifizieren, die die fremde genetische Konstruktion tragen.

7. Verfahren nach Anspruch 6, bei dem das Markergen das Gus-Gen ist.

8. Verfahren nach irgendeinem der vorangegangenen Ansprüche, bei dem man in Stufe (e) den physikalischen Beschleunigungsschritt mit ausreichender Kraft und Intensität durchführt, so daß zwischen 20 und 60 % der embryonischen Achsen derart beschädigt werden, daß sie in Stufe (f) niemals Triebe hervorrufen.

9. Verfahren nach irgendeinem der vorangegangenen Ansprüche, bei dem man in Stufe (e) den physikalischen Beschleunigungsschritt mit ausreichender Kraft und Intensität durchführt, so daß die Mehrzahl an Zellen in den embryonischen Meristemen getötet werden.

10. Verfahren nach irgendeinem der vorangegangenen Ansprüche, bei dem man Stufe (e) durchführt, indem man die beschichteten Trägerpartikel auf einem ebenen Trägerblatt plaziert, man das Trägerblatt an der Trägeroberfläche mit einer Schockwelle einstellbarer Kraft beschleunigt und man das Trägerblatt derart zurückhält, daß die Trägerpartikel auf die Zieloberfläche zuwandern können.

11. Verfahren nach Anspruch 10, bei dem man die Schockwelle einstellbarer Kraft durch eine elektrische Funkenentladung erzeugt und die Kraft dadurch einstellbar ist, daß man die elektrische Spannung der Entladung einstellt.

12. Verfahren nach irgendeinem der vorangegangenen Ansprüche, bei dem der Baumwollsamen einer Sorte entstammt, die aus der Gruppe bestehend aus Pima-, Avala- und Upland-Baumwollsorten ausgewählt ist.

13. Verfahren zur Erzeugung genetisch transformierter Linien von Baumwollpflanzen, bei dem man
(a) Baumwollsamen keimen läßt,
(b) von den keimenden Baumwollsamen embryonische Achsen isoliert,
(c) alle embryonischen Blätter von den embryonischen Achsen entfernt, um die Meristeme der Achsen zu exponieren,
(d) man die embryonischen Achsen zur. Partikelbehandlung vorbehandelt durch Kultivieren der embryonischen Achsen im Dunkeln auf einem Medium mit einem Cytokinin, das aber im wesentlichen Auxin-frei ist,
(e) man die isolierten embryonischen Achsen auf einer Ziel-Oberfläche plaziert,
(f) man Kopien einer fremden genetischen Konstruktion herstellt, die ein interessierendes Fremdgen und ein Markergen einschließt,
(g) man die Kopien der fremden genetischen Konstruktion auf Trägerpartikeln beschichtet, die im Verhältnis zu der Größe der Zellen des Baumwollgewebes klein sind,
(h) die Trägerpartikel, die die Kopien des fremden genetischen Materials an der Zieloberfläche, auf der die embryonischen Achsen plaziert sind, derart physikalisch beschleunigt, daß vielen Trägerpartikel in das Innere der Zellen der embryonischen Achsen hineingeschossen werden,
(i) die embryonischen Achsen wie in Stufe (e) auf einem im wesentlichen Hormon-freien Medium und unter Bedingungen kultiviert, so daß aus wenigstens einigen der behandelten embryonischen Achsen Triebe entstehen, ohne die Kalluskultur zu beschädigen, und man
(j) man die Triebe daraufhin screent, ob sie das Markergen exprimieren, und man
(k) diese Triebe, die die fremde genetische Konstruktion in ihrem Genom enthalten, zu vollständigen, sexuell ausgereiften Baumwollpflanzen kultiviert, die in der Lage sind, das Fremdgen durch sexuelle Vererbung auf ihre Nachfolgepflanzen zu übertragen.

14. Verfahren nach Anspruch 13, bei dem das Markergen das Gus-Gen ist.

15. Verfahren nach Anspruch 13 oder 14, bei dem man in Stufe (h) den physikalischen Beschleunigungsschritt mit ausreichender Kraft und Intensität durchführt, so daß zwischen 20 und 60 % der embryonischen Achsen derart zerstört werden, daß sie in Stufe (f) niemals Triebe hervorrufen.

16. Verfahren nach Anspruch 13, 14 oder 15, bei dem man in Stufe (h) den physikalischen Beschleunigungsschritt mit ausreichender Kraft und Intensität durchführt, so daß die Mehrzahl an Zellen in den Meristemen einer jeden Achse getötet werden.

17. Verfahren nach irgendeinem der Ansprüche 13 bis 16, bei dem man Stufe (h) durchführt, indem man die beschichteten Trägerpartikel auf einem ebenen Trägerblatt plaziert, man das Trägerblatt an der Trägeroberfläche mit einer Schockwelle einstellbarer Kraft beschleunigt und man das Trägerblatt derart zurückhält, daß die Trägerpartikel auf die Zieloberfläche zuwandern können.

18. Verfahren nach Anspruch 17, bei dem man die Schockwelle einstellbarer Kraft durch eine elektrische Funkenentladung erzeugt und die Kraft dadurch einstellbar ist, daß man die elektrische Spannung der Entladung einstellt.

19. Verfahren nach irgendeinem der Ansprüche 13 bis 18, bei dem der Baumwollsamen einer Sorte entstammt, die aus der Gruppe bestehend aus Pima-, Avala- und Upland-Baumwollsorten ausgewählt ist.

20. Verfahren zur Erzeugung genetisch transformierter Baumwollpflanzen-Linien, bei dem man
(a) Baumwollsamen keimen läßt,
(b) von den keimenden Baumwollsamen embryonische Achsen isoliert,
(c) man die isolierten embryonischen Achsen auf einer Zieloberfläche plaziert,
(d) man die Kopien der fremden genetischen Konstruktion auf Trägerpartikeln beschichtet, die im Verhältnis zu der Größe der Zellen des Baumwollgewebes klein sind,
(e) man die Trägerpartikel, die die Kopien des fremden genetischen Materials tragen, an der Zieloberfläche derart physikalisch beschleunigt, daß viele der Trägerpartikel in das Innere der Zellen in den embryonischen Achsen hineintreten,
(f) man die embryonischen Achsen auf einem Medium, das im wesentlichen Hormon-frei ist, derart kultiviert, daß Triebe mit Blättern aus den behandelten embryonischen Achsen hervortreten, ohne die Kalluskultur zu beschädigen,
(g) man ein Blatt von der Pflanze abschneidet und einen Abschnitt des Blattes daraufhin untersucht, ob es das Markergen exprimiert,
(h) die Triebe, bei denen die Untersuchung ergab, daß sie das Markergen in der Mittelrippe des Blattabschnitts exprimieren, zu einer sexuell ausgereiften Pflanze kultiviert,
(i) man den Blütenstaub der Pflanzen daraufhin untersucht, ob er das Markergen exprimiert, um festzustellen, ob die Transformation ein Keimbahn-Ereignis beinhaltet, und man
(j) von denjenigen Pflanzen, die ein Keimbahn-Ereignis beinhalten, die Nachkommen der Pflanzen wiedergewinnt, die die fremde genetische Konstruktion enthalten.

21. Verfahren nach Anspruch 20, bei dem das Markergen das Gus-Gen ist.

## Revendications

1. Méthode pour la création de lignées génétiquement transformées de plants de coton comprenant les étapes de:
(a) faire germer la graine de coton;
(b) isoler les axes embryonnaires de la graine de coton en germination;
(c) placer les axes embryonnaires isolés sur une surface cible;
(d) préparer des copies d'une construction génétique étrangère et enduire les coptes sur des particules porteuses qui sont de petite dimension relativement à la dimension des cellules des tissus du coton;
(e) accélérer physiquement les particules porteuses portant les copies du matériel génétique étranger à la surface cible sur laquelle sont placés les axes embryonnaires de manière que de nombreuses particules porteuses se logent à l'intérieur des cellules des axes embryonnaires;
(f) mettre les axes embryonnaires en culture comme à l'étape (e) sur un milieu sensiblement exempt d'hormones et dans des conditions telles que des pousses proviennent d'au moins certains des axes embryonnaires traités sans culture du calus intermédiaire; et
(g) mettre les pousses en culture qui contiennent la construction génétique étrangère dans leur génome en plants de coton entiers sexuellement mûrs capables de transmettre le gène étranger par héritage sexuel à leur descendance.

2. Méthode selon la revendication 1 où, à l'étape (a) on fait germer la graine jusqu'à ce que un à quatre millimètres du radicule soient exposés.

3. Méthode selon la revendication 1 ou 2, où après avoir isolé les axes embryonnaires de la graine, les axe embryonnaires sont dissectés pour retirer toutes le feuilles embryonnaires pouvant couvrir le méristèmes.

4. Méthode selon la. revendication 1, 2 ou 3 où après l'étape (b), les axes embryonnaires sont prétraités par culture en l'absence sensible de lumière dans un milieu ayant une cytokinine mais pas d'auxine.

5. Méthode selon l'une quelconque des revendications précédentes où après l'étape (a), les axes embryonnaires sont soumis à incubation sur un milieu ayant une cytokine mais pas d'auxine en l'absence sensible de lumière pendant un court temps.

6. Méthode selon l'une quelconque des revendications précédentes où; à l'étape (d) les copies de la construction génétique étrangère contiennent à la fois un gène étranger d'intérêt et un gène marqueur, et où les pousses de l'étape (f) sont criblées pour l'expression du gène marqueur pour indentifier les pousses portant la construction étrangère.

7. Méthode selon la revendiction 6 où le gène marqueur est le gène Gus.

8. Méthode selon l'une quelconque des revendication précédentes où à rétape (e), l'étape d'accélération physique est accomplie avec une force et une intensité suffisantes pour qu'entre 20 et 60 pour cent des axes embryonnaires soient endommagés au point de ne jamais donner de pousse à l'étape (f).

9. Méthode selon l'une quelconque des revendications précédentes où à l'étape (e), l'étape d'accélération physique est accomplie avec une force et une intensité suffisantes pour que la majorité des cellule des méristèmes embryonnaires soient tuées

10. Méthode selon l'une quelconque des revendications précédentes où l'étape (e) est accomplie en plaçant les particules porteuses enduites sur une feuille porteuse plane en accelérant la feuille porteuse à la surface cible avec une onde de choc d'une force réglable et en retenant la feuille porteuse de manière que les particules porteuses puissent se déplacer vers la surface cible.

11. Méthode selon la revendication 10 où l'onde de cho de force réglable est créée par une décharge à étincelle électrique et la force est réglable en ajustan la tension électrique de la décharge.

12. Méthode selon l'une quelconque des revendications précédenes où la graine de coton est d'une variété sélectionnée dans le groupe consistant en Pima, . Avala et Upland Cotton.

13. Méthode pour la création de lignées génétiquement transformées de plants de coton comprenant les étapes de :
(a) faire germer une graine de coton;
(b) isoler les axes embryonnaires de la graine de coton ayant germé;
(c) dissecter toutes les feuilles embryonnaires des axes embryonnaires pour exposer les méristèmes des axes;
(d) prétraiter les axes embryonnaires pour le traltement des particules par mise en culture des axes embryonnaires à l'obscurité sur un milieu avec une cytokinine mais sensiblement exempt d'auxine;
(e) placer les axes embryonnaires isolés sur une surface cible,
(f) préparer des copies d'une construction génétique étrangère comprenant un gène étranger d'intérêt et. un gène marqueur;
(g) enduire les copies de la construction génétique étrangère sur des particules porteuses qui sont d'une petite dimension relativement à la dimension des cellules du tissu de coton;
(h) accélérer physiquement les particules porteuses portant les copies du matériel génétique étranger à la surface cible sur laquelle les axes embryonnaires sont placés de façon que de nombreuses particules porteuses se logent à l'intérieur des cellules des axes embryonnaires;
(i) mettre les axes embryonnaires de l'étape (h) en culture, sur un milieu sensiblement exempt d'hormones et dans des conditions telles que des pousses proviennent d'au moins certains des axés embryonnaires traités sans culture de calus intermédiaire;
(j) cribler les pousses pour l'expression du gène marqueur; et
(k) mettre les pousses en culture qui contiennent la construction génétique étrangère dans leur génome dans des plants de coton entiers sexuellement mûrs capable de transmettre le gène étranger par héritage sexuel à leur descendance.

14. Méthode selon la revendication 13 où le gène marqueur est le gène Gus.

15. Méthode selon la revendication 13 ou 14 où à l'étape (h), l'étape d'accélération physique est accomplie avec une force et une intensité suffisantes pour qu'entre 20 et 60 pour cent des axes embryonnaires soient endommagés de façon à ne jamais donner de pousse à l'étape (i).

16. Méthode selon la revendication 13, 14 ou 15 où à l'étape (h), l'étape d'accélération physique est accomplie avec une force et une intensité suffisantes pour que la majorité des cellules dans le méristème de chaque axe soient tuées.

17. Méthode selon l'une quelconque des revendications 13 à 16 où l'étape (h) est accomplie en plaçant les particules porteuses enduites sur une feuille porteuse plane, en faisant accélérer la feuille porteuse à la surface cible avec une onde de choc d'une force réglable et en retenant la feuille porteuse de manière que les particules porteuses puissent se déplacer vers la surface cible.

18. Méthode selon la revendication 17 où l'onde de choc de force réglable est créée par une décharge à étincelle électrique et la force est réglable en ajustant la tension électrique de la décharge.

19. Méthode selon l'une quelconque des revendications 13 à 18 où la graine de coton est d'une variété sélectionnée dans le groupe consistant en Pima, Acala et Upland Cotton.

20. Méthode de création de lignées génétiquement transformées de plants de coton comprenant les étapes de:
(a) faire germer une graine de coton;
(b) isoler les axes embryonnaires de la graine de coton ayant germé;
(c) placer les axes embryonnaires isolés sur une surface cible;
(d) préparer des copies, d'une construction génétique étrangère comprenant un gène marqueur et enduire les copies sur les particules porteuses qui: sont d'une petite dimension relativement à là dimension des cellules des tissus du coton;
(e) accélérer physiquement les particules porteuses portant les copies du matériel génétique étranger à la surface cible de manière qu'un grand nombre des particules porteuses se logent à l'intérieur des cellules dans les axes embryonnaires;
(f) mettre les axes embryonnaires en culture sur un milieu sensiblement exempt d'hormones de manière que les pousses avec des feuilles proviennent des axes embryonnaires traités sans culture du calus intermédiaire;
(g) couper une feuille du plant et analyser une section de la feuille pour l'expression du gène marqueur;
(h) pour des pousses pour lesquelles l'analyse a révélé l'expression du gène marqueur dans la nervure médiane de la section de la feuille, mettre la pousse en culture dans un plant sexuellement mûr;
(i) analyser le pollen des plants pour l'expression du gène marqueur pour déterminer si la transformation concerne un événemént dans la lignée du germe; et
(j) pour les plants qui ont concerné un événement dans la lignée du germe, récupérer la descendance des plants qui contiennent la construction génétique étrangère.

21. Méthode selon la revendication 20 où le gène marqueur est le gène Gus.
